# FASCICULE DE BREVET EUROPEEN

(11) **EP 2 308 550 B1**
(45) Date de publication et mention de la délivrance du brevet: **13.01.2016**
(21) Numéro de dépôt: 10174448.0
(22) Date de dépôt: 30.08.2010
(51) Int. Cl.: A61N 1/05, A61M 25/00, A61M 25/06

(54) **Sonde épicardique de stimulation/défibrillation à vis, implantable par un cathéter-guide introduit dans l'espace péricardique**
Epikardiale Sonde zur Stimulation/Defibrillation mit einer Schraube, die mit Hilfe eines Führungskatheters in den perikardialen Raum implantiert werden kann
Epicardial stimulation/defibrillation probe with screw, suitable for implantation via a catheter-guide inserted in the pericardial cavity

(30) Priorité: 07.10.2009 FR 0956982
(43) Date de publication de la demande: 13.04.2011
(73) Titulaire: Sorin CRM SAS, 92541 Montrouge (FR)
(72) Inventeur: Ollivier, Jean-François, 91190, Villiers le Bacle (FR)
(74) Mandataire: Dupuis-Latour, Dominique

(56) Documents cités:
- WO-A2-03/086502
- WO-A2-2006/116719
- WO-A2-2007/115158
- US-A- 5 330 496
- US-A1- 2007 100 409
- US-A1- 2008 208 166

## Description

L'invention concerne les "dispositifs médicaux implantables actifs" tels que définis par la directive 90/385/CEE du 20 juin 1990 du Conseil des communautés européennes, notamment les implants permettant de surveiller en continu le rythme cardiaque et délivrer si nécessaire au coeur des impulsions électriques de stimulation, de resynchronisation, ou de défibrillation.

Elle concerne plus précisément les sondes dites "épicardiques" qui sont fixées sur la paroi extérieure du coeur, par opposition aux sondes dites "endocardiaques" qui sont logées dans une cavité du coeur où elles y sont introduites via le réseau veineux.

On fera surtout référence dans la suite aux sondes de stimulation, c'est-à-dire de délivrance d'impulsions à basse énergie, utilisées pour les thérapies antibradycardiques ou de resynchronisation. Mais l'invention s'applique également aux sondes épicardiques de cardioversion/défibrillation destinées à délivrer au coeur des chocs électriques de haute énergie pour tenter de mettre fin à une tachyarythmie. On utilisera le terme générique de "sonde (ou électrode) de stimulation/défibrillation" pour désigner tout type de sonde utilisable à ces fins.

Les sondes épicardiques peuvent être notamment prescrites pour la stimulation du ventricule gauche, en alternative aux sondes de stimulation implantées via le sinus coronaire, qui nécessitent pour leur pose une approche délicate et ne sont pas dénuées d'inconvénients divers.

A la différence des sondes endocavitaires introduites via le réseau veineux, la mise en place d'une sonde épicardique est une opération très invasive, nécessitant généralement une anesthésie générale et le recours à des techniques chirurgicales. Il est en effet nécessaire qu'un chirurgien incise le thorax de manière à ménager un accès au "sac péricardique" (le péricarde étant l'enveloppe fibro-séreuse qui enveloppe le coeur), puis au myocarde lui-même, afin de pouvoir fixer la sonde sur la paroi externe de ce dernier, par suturation ou par vissage.

Plusieurs types de sondes épicardiques ont été proposées, mais toutes présentent un certain nombre d'inconvénients assez sérieux.

Les sondes suturées sont très stables, mais elles nécessitent un large accès (pour permettre la suture par le chirurgien), et la zone de pose possible, restreinte à la proximité de l'incision thoracique, est très limitée.

On a également proposé des sondes à visser. Le vissage peut se faire directement, mais la zone de travail sera limitée de la même façon que pour une sonde suturée. Il peut également se faire au moyen d'un instrument de pose comprenant un manche, un tube support télescopique et une tête articulée sur laquelle est montée la sonde. Dans ce dernier cas, le chirurgien pourra accéder à des sites situés au-delà de l'incision, mais la zone de pose possible sera toutefois limitée par la rigidité du tube support de l'instrument, et par le diamètre important de ce tube (de l'ordre de 40 French), que le médecin doit faire progresser dans l'espace péricardique courbe.

En tout état de cause, les sondes à vis proposées jusqu'à présent présentent de faibles performances électriques en raison de la taille importante de la vis (nécessaire pour supporter les conditions de pose mais qui ne procure pas une densité de courant satisfaisante). De plus, ces vis de fixation sont relativement traumatiques pour les tissus, avec risque de création localement de fibroses réactionnelles.

Il est toutefois important, sur le plan mécanique, que les vis des sondes actuelles soient surdimensionnées, en raison des contraintes élevées exercées lors de la pose sur la vis, avec des accessoires très volumineux et offrant peu de sensibilité. La tête de sonde y est soumise à des contraintes mécaniques importantes dues à de fortes amplitudes de déplacement, combinées aux tractions radiales exercées via le corps de sonde. L'ancrage mécanique de la tête de sonde sur le myocarde doit donc être capable de résister à ces diverses contraintes.

Une autre caractéristique commune à la quasi-totalité des sondes épicardiques actuelles est le fait que le corps de sonde (la gaine reliant la tête de sonde au générateur de stimulation) se raccorde à angle droit à la tête de sonde, c'est-à-dire que l'axe de la gaine est perpendiculaire à l'axe de la vis. Ceci donne à l'ensemble une configuration volumineuse, donc traumatique dans la région du site de stimulation.

Enfin, la géométrie de ces sondes rend impossible leur retrait par simple vissage et nécessite une intervention lourde.

Le but de l'invention est de proposer un nouveau type de sonde épicardique, et un accessoire de pose de cette sonde, qui pallient l'ensemble des inconvénients évoqués ci-dessus, en permettant notamment de :
- limiter la dimension de l'incision pour l'accès au coeur, celle-ci pouvant être réduite, comme on le verra, à une simple ponction ;
- miniaturiser la tête de sonde pour la rendre moins traumatique ;
- miniaturiser l'accessoire de pose, de manière à offrir une plus grande sensibilité au praticien et rendre l'intervention moins invasive ;
- maximiser la zone de travail autour du point d'entrée dans l'enveloppe péricardique, et donc l'étendue des sites de stimulation potentiels ;
- augmenter la manoeuvrabilité pour le choix du site ;
- permettre une voie d'accès ne nécessitant pas une tunnellisation ;
- offrir une possibilité de "mapping" avant le vissage définitif de la sonde, c'est-à-dire de test de la réponse électrique du site d'implantation envisagé ;
- assurer sur le plan mécanique une fixation de qualité, mais moins traumatique pour les tissus ;
- accroître les performances électriques ;
- autoriser une extraction par des moyens simples ;
- offrir une configuration de type "isodiamètre" (ou "monodiamètre") compatible avec les techniques chirurgicales d'implantation courantes.

Le principe de base de l'invention consiste à choisir comme sonde épicardique une sonde à vis, mais dont la vis s'étend dans le prolongement du corps de sonde (et non plus à 90° par rapport à ce dernier), avec une vis de taille et de caractéristiques comparables à celles des sondes endocavitaires à vis.

L'invention propose en outre, pour la mise en place d'une telle sonde sur la paroi externe du myocarde, d'utiliser comme outil de pose un cathéter-guide dans lequel sera glissée la sonde. Ce cathéter-guide pourra être introduit dans l'espace péricardique par une simple ponction (donc sans incision de l'enveloppe péricardique), et il sera possible de le faire évoluer autour de la surface, approximativement sphérique, du myocarde, pour "naviguer" ainsi sur l'épicarde en toute sécurité (du fait de la flexibilité du cathéter-guide et de son embout *soft tip* atraumatique) jusqu'au site de stimulation choisi, qui pourra être très éloigné de la ponction constituant le point d'accès à l'espace péricardique.

Le US 2008/0208166 A1 décrit un cathéter endocardiaque de délivrance préformé, avec une double courbure formant un *heart-wrapping segment* destiné à envelopper la surface extérieure du coeur en englobant ce dernier sur la majeure partie de l'épicarde.

Un tel cathéter, s'il était combiné à une sonde à vis, permettrait certes le vissage mais limiterait très sensiblement les capacités d'évoluer sur une large région à la surface du coeur. En effet, les larges courbes du *heart-wrapping* tendent en permanence à coïncider avec la forme externe du coeur, par le principe de moindre énergie. Il en résulte une position d'équilibre donnée (donc un site d'implantation donné) pour une courbure donnée, d'où la nécessité de plusieurs modèles (différentes courbures) si l'on veut plusieurs sites d'implantation possibles.

La divulgation du US 2008/0208166 A1 laisse donc subsister le besoin d'un cathéter-guide permettant :
- de naviguer librement dans l'espace péricardique sans être gêné par le principe de moindre énergie ;
- une fois le site choisi, d'orienter l'extrémité de la courbure d'appui dans la bonne direction (en ne laissant la possibilité que de deux positions angulaires stables facilement différenciables, à savoir : vers l'épicarde ou vers le sac péricardique) ; et
- de sécuriser la position retenue par un couple manuel exercé côté proximal.

Une autre technique encore, décrite par le WO 03/086502 A2, met en oeuvre un fil-guide préalablement introduit dans l'espace péricardique, et ancré à son extrémité distale dans la paroi du myocarde. Une sonde pourvue d'électrodes surfaciques est alors enfilée sur ce fil-guide jusqu'au site d'implantation choisi.

Si l'on veut combiner un cathéter-guide introduit dans l'espace péricardique avec une sonde à vis, le problème est dans tous les cas d'assurer une stabilisation du cathéter-guide une fois le site choisi, de manière à permettre le vissage de la tête de sonde pour fixer celle-ci définitivement. Pour cela, selon l'invention, le cathéter-guide est un cathéter préformé avec deux courbures successives dans sa région distale :
- la première courbure (la plus éloignée de l'extrémité du cathéter-guide) est une courbure d'appui permettant au cathéter-guide de s'appuyer sur la paroi extérieure du sac péricardique sous l'effet d'un couple de torsion imprimé par le praticien, et permettant de stabiliser en place le cathéter-guide en dépit des sollicitations diverses notamment sous l'effet des battements du coeur ;
- l'autre courbure, située à proximité de l'extrémité distale du cathéter-guide, est une courbure d'orientation permettant de tourner l'axe de la vis d'ancrage vers la paroi externe du myocarde, en formant avec cette dernière un angle suffisant pour y permettre le vissage selon un angle d'attaque prédéfini.

Les faibles dimensions de la sonde et du cathéter-guide (typiquement de l'ordre de 6 à 9 French), combinées à l'isodiamétrie de la sonde, font qu'il suffit d'une simple ponction pour introduire le cathéter-guide et la sonde, sans incision du sac péricardique.

D'autre part, le caractère monodiamètre de la sonde est très favorable à l'extraction, qui pourra être opérée simplement par dévissage et retrait, sans intervention chirurgicale lourde.

Plus précisément, l'ensemble de l'invention comprend, de manière en elle-même connue, les éléments énoncés au préambule de la revendication 1, connus d'après le WO 2003/086502 A2 précité.

De façon caractéristique de l'invention, l'ensemble comprend les éléments énoncés dans la partie caractérisante de la revendication 1.

Les sous-revendications visent des formes de réalisation préférentielles, avantageuses.

On va maintenant décrire un exemple de réalisation de l'invention, en référence aux dessins annexés où les mêmes références numériques désignent d'une figure à l'autre des éléments identiques ou fonctionnellement semblables.
La Figure 1 est une vue en coupe de la tête de sonde de l'ensemble selon l'invention.
La Figure 2 illustre schématiquement la configuration de l'ensemble constitué par la sonde de la Figure 1, combinée à un cathéter-guide à double courbure et à un mandrin introduit dans la sonde, cette illustration étant vue en perspective montrant la forme de l'ensemble dans l'espace.
La Figure 3 est une vue partielle en coupe montrant les différentes tuniques du coeur et illustrant la manière dont l'ensemble cathéter-sonde selon l'invention est introduit et positionné dans le sac péricardique.
La Figure 4 est une vue en coupe de la sonde de la Figure 1 après que celle-ci ait été implantée dans l'espace péricardique, avec la vis ancrée dans la paroi externe du myocarde.
La Figure 5 est une vue de bout selon V-V de la Figure 2.
La Figure 6 est une vue de dessus, selon VI-VI de la Figure 2.
◊

On va maintenant décrire un exemple de réalisation de l'invention.

Sur les figures, la référence 10 désigne de façon générale la sonde à vis de l'ensemble selon l'invention. Cette sonde 10 comporte un corps de sonde 12 de structure en elle-même connue, généralement une gaine en polyuréthanne pour limiter les frottements lorsque la sonde est introduite dans un cathéter-guide, et pour procurer une meilleure sensibilité et une meilleure transmission du couple de torsion. Le diamètre de la gaine du corps de sonde 12 est choisi suffisamment fin (typiquement inférieur ou égal à 5 French) pour être compatible avec les dimensions de l'espace péricardique et avec le diamètre des cathéters-guides usuels.

La sonde 10 est terminée à son extrémité distale par une vis d'ancrage hélicoïdale 14 en matériau conducteur, reliée par l'intermédiaire d'un embout métallique 16 à un conducteur interne 18 tel qu'un conducteur spiralé assurant la continuité électrique entre la vis d'ancrage, qui fait office d'électrode de stimulation et/ou de recueil, et le générateur situé à l'extrémité proximale (non représentée) de la sonde. On notera que la gaine en polyuréthanne peut être choisie pour procurer une certaine rigidité en torsion au corps de sonde 12, de manière à pouvoir transmettre un couple de torsion depuis l'extrémité proximale jusqu'à l'extrémité distale afin d'entraîner en rotation la vis 14 pour la faire pénétrer par vissage dans le tissu cardiaque.

A la différence des sondes épicardiques classiques où la vis s'étend à angle droit par rapport au corps de sonde auquel elle est raccordée, la vis d'ancrage 14 est ici une vis axiale, c'est-à-dire qu'elle s'étend dans le prolongement du corps de sonde 12, procurant ainsi à l'ensemble une configuration d'ensemble de type "isodiamètre" ou "monodiamètre".

La vis 14 est avantageusement réalisée avec une partie distale formée, sur une longueur de l'ordre de 1,5 à 2 mm, de spires non jointives 20 reliées au corps de sonde par l'intermédiaire d'une partie de transition mécanique présentant une souplesse en flexion, par exemple une partie formée de spires 22 jointives en l'absence de sollicitation de la vis.

La vis 14 est avantageusement isolée sur toute sa longueur, par exemple par un revêtement de parylène, à l'exception du dernier millimètre distal qui sera donc la seule partie électriquement active de la vis, ce qui permet de diminuer la surface de stimulation et accroître d'autant la densité de courant et donc l'efficacité électrique de la sonde. Cette partie électriquement active sera en outre enfouie assez profondément dans la paroi de l'épicarde, ce qui permet d'y concentrer le flux électrique et de stimuler une zone profonde, plus physiologique, en limitant de surcroît le risque de stimulation phrénique.

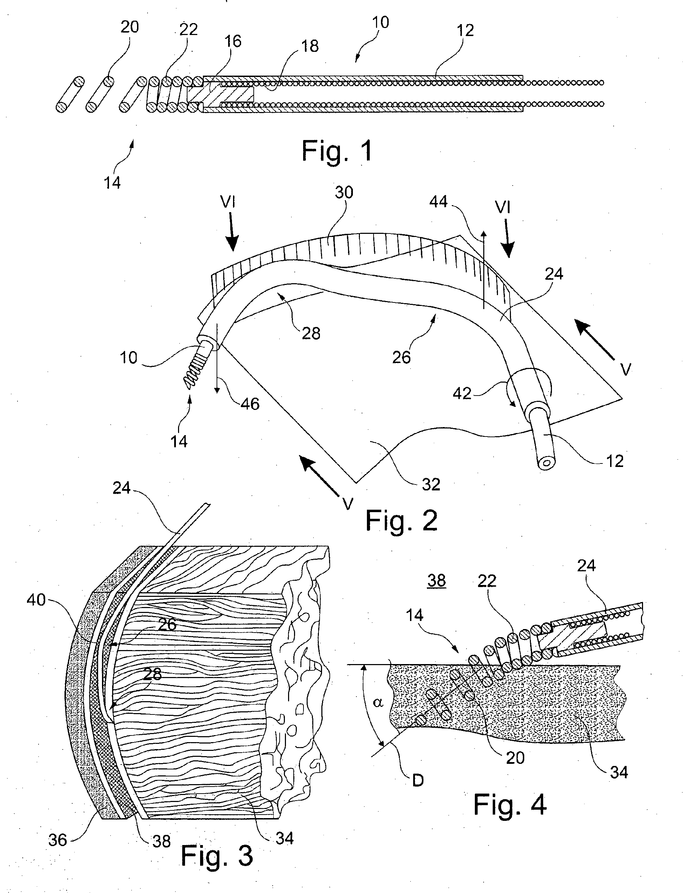

Dans le mode de réalisation illustré, la vis 14 est une vis active, c'est-à-dire jouant (au moins à son extrémité distale) le rôle d'électrode de stimulation. En variante, la vis peut être une vis électriquement passive ne servant qu'à l'ancrage de la tête de sonde contre la paroi de l'épicarde, la tête de sonde étant dans ce dernier cas pourvue à son extrémité d'une électrode distale spécifique, par exemple en forme d'anneau de stimulation. Sur la Figure 2, on a illustré le cathéter-guide 24 utilisé pour l'implantation de la sonde au site de stimulation choisi, avec la sonde 10 introduite à l'intérieur. La partie distale de ce cathéter-guide 24 est ouverte à son extrémité, de manière à pouvoir faire émerger l'extrémité distale de la sonde 10 et sa vis d'ancrage 14 par déplacement axial relatif du corps de sonde 12 à l'intérieur du cathéter-guide 24.

Ce cathéter-guide 24 présente à sa partie distale une double courbure 26, 28, chacune de ces courbures étant inscrite dans une surface distincte respective 30, 32.

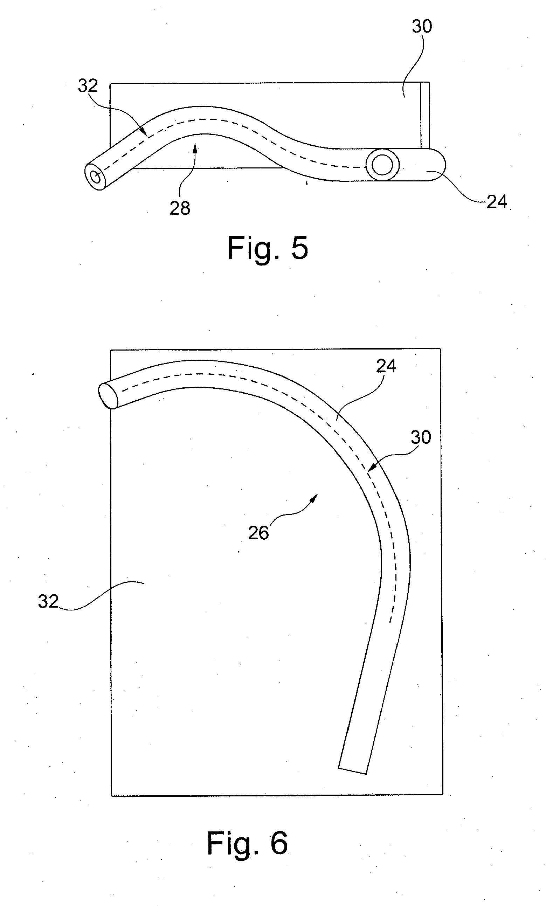

La surface curviligne 30 définit une courbure 26 (ci-après "courbure d'appui") qui non seulement permet de suivre naturellement la courbure relativement sphérique lors de la progression du cathéter-guide dans l'espace péricardique, mais qui permet également, lorsque le site d'implantation a été choisi, de créer un appui contre la paroi intérieure du sac péricardique de manière à maintenir en place le cathéter-guide dans ce dernier en dépit des battements cardiaques. Cette partie curviligne 30 présente une dimension hors-tout en plan (correspondant à la vue de dessus de la Figure 6) de l'ordre de 15-25 mm sur 10-15 mm.

La surface curviligne 32, quant à elle, définit une courbure 28 (ci-après "courbure d'orientation") permettant de diriger la tête de sonde, et donc l'axe de la vis d'ancrage, vers la paroi du myocarde selon un angle d'attaque prédéfini. Cette partie curviligne 32 présente une dimension hors-tout en élévation (correspondant à la vue de bout de la Figure 5) de l'ordre de 10-15 mm sur 5 mm au plus.

En d'autres termes, la double courbure 26, 28 s'inscrit dans un volume parallélépipédique de dimensions comprises entre 15 x 10 x 5 mm et 25 x 15 x 5 mm, dimensions compatibles avec le positionnement dans l'espace péricardique et les fonctions recherchées de maintien et de guidage de la tête de sonde.

La référence 34 désigne le myocarde proprement dit, c'est-à-dire le muscle cardiaque, qui est enveloppé dans un sac fibro-séreux ou "sac péricardique" 36 définissant entre la paroi externe du myocarde et sa paroi interne un espace péricardique 38, rempli d'une faible quantité de liquide et permettant de faciliter les mouvements du coeur à l'intérieur du thorax.

Le diamètre du cathéter-guide est choisi suffisamment fin, typiquement 9 French, voire 6 French, pour permettre une navigation aisée dans l'espace péricardique 38. Il est également possible d'utiliser un cathéter-guide 6 French renforcé par un cathéter-guide 9 French standard, l'ensemble assurant une fonction télescopique et permettant d'augmenter la rigidité du corps pour en améliorer le contrôle, selon une procédure en elle-même classique.

Une fois le site de stimulation identifié, le praticien maintient le cathéter-guide 24 en position en appliquant un couple de torsion sur une poignée (non représentée) située à l'extrémité proximale du cathéter-guide. Du fait de la courbure 26, le cathéter-guide 24 vient alors en appui en 40 contre la paroi externe de l'espace péricardique 38, générant ainsi un couple résistant en sens inverse qui contraint en place l'extrémité distale du cathéter-guide, et donc de la sonde, qui se trouve alors maintenue en appui sur le site d'implantation choisi, tout en suivant les mouvements des battements cardiaques.

La courbure d'orientation 28 à l'extrémité distale a pour effet de diriger l'axe de la vis d'ancrage 14 non pas dans le prolongement de l'espace péricardique 38, mais au contraire, comme illustré Figure 4 (après retrait du cathéter-guide) vers la paroi externe du myocarde 34 avec un angle d'attaque prédéterminé. De cette manière, l'accrochage de la vis et le vissage subséquent de celle-ci seront guidés suivant une direction D faisant un angle α avec la direction générale axiale de la sonde.

Une fois le site atteint, le praticien fait coulisser la sonde 10 à l'intérieur du cathéter-guide 24, jusqu'à ce que l'extrémité distale de cette sonde et sa vis d'ancrage 14 émergent de l'extrémité correspondante du cathéter-guide (configuration illustrée Figure 2). Grâce à la double courbure décrite plus haut de l'extrémité distale du cathéter-guide 24, l'orifice distal du cathéter-guide est dirigé vers la paroi externe du myocarde 34 en formant un angle α par rapport à la direction générale de l'espace péricardique. Il suffit alors d'avancer dans ce cathéter-guide la sonde 10 jusqu'à ce qu'elle débouche du cathéter-guide pour que sa vis d'ancrage vienne en contact avec cette paroi 34.

Il est alors possible de procéder à un *mapping* préalable pour tester électriquement le point de contact et valider le site de stimulation choisi. Si la position n'est pas satisfaisante, il suffit au praticien de déplacer sous contrôle le cathéter-guide dans le sac péricardique vers un autre point et de tester un nouveau site.

L'ancrage définitif est obtenu en imprimant un mouvement de rotation axiale au corps de sonde (dans le cas d'une sonde à vis fixe) ou à la fiche du connecteur (pour une sonde de type *pin driven,* où du côté proximal la fiche de connexion est solidaire d'un conducteur axial s'étendant à l'intérieur du corps de sonde, ce conducteur étant lui-même libre en rotation et relié à la vis d'ancrage à son extrémité distale). Une autre possibilité encore consiste à introduire dans la lumière du corps de sonde un mandrin de vissage spécifique, ceci notamment lorsque la gaine ne présente pas une rigidité en torsion suffisante pour entraîner la vis directement depuis l'extrémité proximale.

En variante, au lieu d'une vis fixe, il est possible d'utiliser une vis de type vis rétractable, avec un mécanisme en lui-même connu. Dans ce cas, le mouvement de rotation entraîne tout d'abord le déploiement de la vis hors de son logement, puis dans un second temps la pénétration de celle-ci dans la paroi du myocarde.

La dernière étape consiste à retirer le cathéter-guide 24, ce qui est fait selon une procédure classique de découpe au moyen d'un outil de type *slitter* tel que celui décrit dans le EP 2 039 390 A1 (ELA Medical).

La configuration finale, et définitive, est celle illustrée Figure 4.

La tête de la sonde n'étant plus maintenue par le cathéter-guide porteur, elle s'articule alors en direction du corps de sonde, grâce à la partie de transition flexible 22, limitant ainsi au maximum l'emprise dans le sens de l'épaisseur de l'espace péricardique.

Parmi les avantages de la technique que l'on vient d'exposer, on peut citer en particulier :
- technique très faiblement invasive : l'accès au sac péricardique ne se fait pas par une incision, mais par une simple ponction, c'est-à-dire un simple trou (dimension 9 French) réalisé à l'aiguille puis élargi, donc qui se refermera lui-même sans qu'il soit nécessaire de procéder à une suture et avec diminution des risques infectieux. Cette intervention légère peut être réalisée par un électrophysiologiste, sans recours à un chirurgien ;
- possibilité de mapper une large zone autour du point d'accès, et même dans des régions très éloignées, voire opposées de ce point d'accès, la navigation du cathéter-guide étant possible dans une très vaste étendue du sac péricardique ;
- la progression dans l'espace péricardique reste très atraumatique par rapport aux solutions actuelles : en effet, la recherche du site optimal se fait avec un cathéter-guide sécurisé par une sonde d'électrophysiologie, ou par un système classique dilatateur souple + fil-guide, à la différence des sondes actuelles où c'est la vis définitive - très agressive - qui est exposée en partie distale de l'accessoire de pose, avec risque de dissection du sac péricardique et/ou des tissus de surface. La navigation est en outre largement facilitée par la faible taille du cathéter-guide, typiquement un diamètre de 6 French ;
- performances électriques supérieures à celles des sondes proposées jusqu'à présent, grâce au découplage mécanique entre le corps de sonde et la vis du fait de la partie de transition flexible ;
- concentration du flux électrique dans une région profonde du myocarde ;
- excellente capacité d'extraction, par simple dévissage de l'extrémité distale : en particulier le caractère isodiamètre de la sonde facilite une telle extraction simplement par dévissage puis retrait ;
- simplicité mécanique d'ensemble, donc faible coût de fabrication et fiabilité élevée.

## Revendications

1. Un ensemble de sonde épicardique de stimulation/défibrillation, pour la stimulation/défibrillation d'une cavité cardiaque en combinaison avec un générateur d'un dispositif médical implantable actif, notamment un stimulateur cardiaque, resynchroniseur ou défibrillateur, cet ensemble comprenant :
- une sonde épicardique de stimulation/défibrillation (10) de type sonde à vis, comportant :
• un corps de sonde (12) avec une gaine en matériau déformable ;
• côté distal, une tête de sonde avec une électrode de stimulation/défibrillation et une vis d'ancrage (14) hélicoïdale saillante apte à pénétrer dans la paroi externe du myocarde (34) sous l'effet d'un mouvement de vissage imprimé à la tête de sonde ; et
• côté proximal, des moyens de couplage au générateur du dispositif médical, et
- un cathéter-guide amovible (24) de pose de la sonde épicardique, apte à être introduit dans l'espace péricardique (38),
ce cathéter-guide comprenant un tube creux ouvert à ses deux extrémités avec une lumière centrale à l'intérieur de laquelle la sonde est apte à être introduite et déplacée en translation à l'intérieur du cathéter-guide, d'abord jusqu'à une position rétractée où la tête de sonde affleure le débouché côté distal de la lumière du cathéter-guide, puis jusqu'à une position déployée où la tête de sonde, avec la vis d'ancrage, émerge de ce débouché, et
le tube creux du cathéter-guide étant un tube préformé, élastiquement déformable et relativement plus rigide que la gaine du corps de sonde, et présentant en l'absence de sollicitation deux courbures successives (26, 28) inscrites dans deux surfaces respectives (30, 32) distinctes, cet ensemble étant **caractérisé en ce que** :
- la sonde (10) est une sonde monodiamètre, avec la vis d'ancrage (14) hélicoïdale prolongeant axialement la tête de sonde ;
- lesdites deux courbures successives (26, 28) s'inscrivent dans un volume parallélépipédique de dimensions comprises entre 15x10x5 mm et 25x15x5 mm ;
- la courbure la moins distale (26) desdites deux courbures successives (26, 28) est une courbure présentant une dimension hors-tout en plan de 15 à 25 mm sur 10 à 15 mm,
de manière à suivre la courbure naturelle de l'espace péricardique lors de la progression du cathéter-guide et à procurer, une fois le site d'implantation choisi, un appui du corps de sonde contre la paroi externe de l'espace péricardique ; et
- la courbure la plus distale (28) desdites deux courbures successives (26, 28) est une courbure présentant une dimension hors-tout en élévation de 10 à 15 mm sur 5 mm au plus,
de manière à permettre, une fois le site d'implantation choisi, une orientation de l'extrémité du tube de cathéter-guide selon un angle d'attaque prédéfini dans la direction de la paroi externe du myocarde (34), et un maintien de l'axe (D) de la vis d'ancrage (14) dans cette même direction lors d'un mouvement combiné de vissage et de déplacement de la tête de sonde vers la position déployée.

2. L'ensemble de la revendication 1, dans lequel la vis d'ancrage (14) est une vis fixe.

3. L'ensemble de la revendication 1, dans lequel la vis d'ancrage est une vis mobile, rétractable dans un logement de la tête de sonde.

4. L'ensemble de la revendication 1, dans lequel la vis d'ancrage (14) est une vis active formant ladite électrode de stimulation/défibrillation.

5. L'ensemble de la revendication 1, dans lequel la vis d'ancrage est une vis passive, électriquement découplée de ladite électrode de stimulation/défibrillation.

6. L'ensemble de la revendication 1, dans lequel la gaine du corps de sonde (12) présente en torsion une rigidité suffisante pour permettre la transmission sur toute sa longueur d'un mouvement de rotation imprimé depuis l'extrémité proximale de la sonde, pour le vissage de la vis d'ancrage.

7. L'ensemble de la revendication 1, dans lequel la gaine du corps de sonde (12) présente en torsion une rigidité insuffisante pour permettre la transmission sur toute sa longueur d'un mouvement de rotation imprimé depuis l'extrémité proximale de la sonde, et dans lequel l'ensemble comprend en outre :
- un mandrin amovible, apte à être introduit dans une lumière du corps de sonde et mobile en translation à l'intérieur de cette lumière jusqu'à la tête de sonde et comportant des moyens de couplage en rotation avec la tête de sonde, ce mandrin présentant en torsion une rigidité suffisante pour permettre la transmission, sur toute sa longueur et jusqu'aux moyens de couplage, d'un mouvement de rotation imprimé depuis l'extrémité proximale du mandrin, pour le vissage de la vis d'ancrage.

8. L'ensemble de la revendication 1, dans lequel le diamètre du cathéter-guide (24) est inférieur ou égal à 9 French, de préférence inférieur ou égal à 6 French.

9. L'ensemble de la revendication 1, dans lequel le diamètre du corps de sonde (12) est inférieur ou égal à 5 French.

10. L'ensemble de la revendication 1, dans lequel la vis d'ancrage (14) comprend une partie d'extrémité (20) apte à pénétrer dans la paroi externe du myocarde (34), et reliée à la tête de sonde par une partie de transition (22) mécaniquement déformable en flexion.

11. L'ensemble de la revendication 10, dans lequel la partie de transition (22) mécaniquement déformable en flexion est une partie à spires jointives de la vis hélicoïdale.

12. L'ensemble de la revendication 1, dans lequel la vis d'ancrage comprend une partie d'extrémité (20) apte à pénétrer dans la paroi externe du myocarde (34), et comprenant elle-même une région active de pointe électriquement conductrice prolongeant une partie intermédiaire électriquement isolée.

13. L'ensemble de la revendication 12, dans lequel la longueur axiale de la région active de pointe électriquement conductrice est d'au plus 1 mm.

## Patentansprüche

1. Anordnung einer epikardialen Sonde zur Stimulation/Defibrilation für die Stimulation/Defibrillation eines Herzraums in Kombination mit einem Generator einer aktiven implantierbaren medizinischen Vorrichtung, insbesondere eines Herzschrittmachers, Resynchronisierers, Defibrillators, wobei die Anordnung Folgendes aufweist:
- eine epikardiale Sonde zur Stimulation/Defibrilation (10) vom Typ der Sonde mit einer Schraube, umfassend:
- einen Sondenkörper (12) mit einer Hülle aus deformierbarem Material;
- auf der distalen Seite einen Sondenkopf mit einer Stimulations-/Defibrillationselektrode und einer hervorstehenden, spiralförmigen Ankerungsschraube (14), die geeignet ist, in die Außenwand des Myokards (34) unter der Wirkung einer Schraubbewegung, die auf den Sondenkopf auferlegt wird, einzudringen; und
- auf der proximalen Seite Mittel zum Koppeln an den Generator der medizinischen Vorrichtung und
- einen entfernbaren Führungskatheter (24) zum Legen der epikardialen Sonde, der geeignet ist, in den perikardialen Raum (38) eingeführt zu werden,
wobei dieser Führungskatheter einen hohlen Schlauch, der an seinen zwei Enden offen ist, mit einem zentralen Lumen aufweist, in dessen Inneres die Sonde geeignet ist, eingeführt zu werden und im Inneren des Führungskatheters zunächst bis zu einer eingefahrenen Position, in der der Sondenkopf mit der Mündung auf der distalen Seite des Lumens des Führungskatheters bündig ist, und dann bis zu einer ausgefahrenen Position, in der der Sondenkopf mit der Ankerschraube aus dieser Mündung herausragt, translatorisch bewegt zu werden, und
wobei der hohle Schlauch des Führungskatheters ein vorgeformter Schlauch ist, der elastisch verformbar und relativ steifer als die Hülle des Sondenkörpers ist, und in Abwesenheit von Beanspruchung zwei aufeinanderfolgende Krümmungen (26, 28) aufweist, die in zwei jeweils getrennten Flächen (30, 32) beschrieben sind,
wobei diese Anordnung **dadurch gekennzeichnet ist, dass**:
- die Sonde (10) eine Sonde vom Typ Monodurchmesser ist, wobei die spiralförmige Ankerungsschraube (14) den Sondenkopf axial verlängert;
- die aufeinanderfolgenden Krümmungen (26, 28) ein quaderförmiges Volumen von Abmessungen zwischen 15 x 10 x 5 mm und 25 x 15 x 5 mm beschreiben;
- die am wenigsten distale Krümmung (26) der zwei aufeinanderfolgenden Krümmungen (26, 28) eine Krümmung ist, die ein Außenmaß in der Ebene von 15 bis 25 mm mal 10 bis 15 mm aufweist,
um der natürlichen Krümmung des perikardialen Raums während des Vordringens des Führungskatheters zu folgen und dem Sonderkörper, nachdem die Implantationsstelle gewählt worden ist, eine Auflage gegen die Außenwand des perikardialen Raums bereitzustellen; und
- die distalste Krümmung (28) der zwei aufeinanderfolgenden Krümmungen (26, 28) eine Krümmung ist, die ein Außenmaß in der Höhe von nicht mehr als 10 bis 15 mm mal 5 mm aufweist,
um, nachdem die Implantationsstelle gewählt worden ist, eine Ausrichtung des Endes des Schlauchs des Führungskatheters nach einem vorbestimmten Anstellwinkel in der Richtung der Außenwand des Myokards (34) und ein Halten der Achse (D) der Ankerschraube (14) in dieser gleichen Richtung bei eine kombinierten Bewegung des Schraubens und des Verschiebens des Sondenkopfes in Richtung der ausgefahrenen Position zu ermöglichen.

2. Anordnung nach Anspruch 1, wobei die Ankerschraube (14) eine feste Schraube ist.

3. Anordnung nach Anspruch 1, wobei die Ankerschraube eine bewegliche Schraube ist, die in ein Gehäuse des Sondenkopfes einziehbar ist.

4. Anordnung nach Anspruch 1, wobei die Ankerschraube (14) eine aktive Schraube ist, die die Stimulations-/Defibrillationselektrode bildet.

5. Anordnung nach Anspruch 1, wobei die Ankerschraube eine passive Schraube ist, die elektrisch von der Stimulations-/Defibrillationselektrode entkoppelt ist.

6. Anordnung nach Anspruch 1, wobei die Hülle des Sondenkörpers (12) in Verdrehung eine Steifigkeit aufweist, die ausreichend ist, um die Übertragung einer Drehbewegung über ihre ganze Länge zu ermöglichen, die von dem proximale Ende der Sonde für das Verschrauben der Ankerschraube auferlegt wird.

7. Anordnung nach Anspruch 1, wobei die Hülle des Sondenkörpers (12) in Verdrehung eine Steifigkeit aufweist, die nicht ausreichend ist, um die Übertragung einer Drehbewegung über ihre ganze Länge zu ermöglichen, die von dem proximale Ende der Sonde auferlegt wird, und wobei die Anordnung ferner Folgendes aufweist:
- einen entfernbaren Dorn, der geeignet ist, in ein Lumen des Sondenkörpers eingeführt zu werden und im Inneren dieses Lumens bis zu dem Sondenkopf verschiebbar ist und Mittel zum Koppeln in Drehung mit dem Sondenkopf aufweist, wobei dieser Dorn in Verdrehung eine Steifigkeit aufweist, die ausreichend ist, um die Übertragung einer Drehbewegung über seine ganze Länge und bis zu den Mitteln zum Koppeln zu ermöglichen, die von dem proximale Ende des Dorns für das Verschrauben der Ankerschraube auferlegt wird.

8. Anordnung nach Anspruch 1, wobei der Durchmesser des Führungskatheters (24) kleiner als oder gleich 9 French, vorzugsweise kleiner als oder gleich 6 French ist.

9. Anordnung nach Anspruch 1, wobei der Durchmesser des Sondenkörpers (12) kleiner als oder gleich 5 French ist.

10. Anordnung nach Anspruch 1, wobei die Ankerschraube (14) einen Endabschnitt (20) aufweist, der geeignet ist, in die Außenwand des Myokards (34) einzudringen, und mit dem Sondenkopf durch einen Übergangsabschnitt (22) verbunden ist, der mechanisch in Biegung verformbar ist.

11. Anordnung nach Anspruch 10, wobei der Übergangsabschnitt (22), der mechanisch in Biegung verformbar ist, ein Abschnitt mit übereinanderliegenden Windungen der spiralförmigen Schraube ist.

12. Anordnung nach Anspruch 1, wobei die Ankerschraube einen Endabschnitt (20) aufweist, der geeignet ist, in die Außenwand des Myokards (34) einzudringen, und selbst einen elektrisch leitenden, aktiven Spitzenbereich aufweist, der einen elektrisch isolierten Zwischenabschnitt verlängert.

13. Anordnung nach Anspruch 12, wobei die axiale Länge des elektrisch leitenden, aktiven Spitzenbereichs mindestens 1 mm beträgt.

## Claims

1. A stimulation/defibrillation epicardial lead unit, for the stimulation/ defibrillation of a heart cavity in combination with a generator of an active implantable medical device, in particular a pacemaker, a resynchronizer or a defibrillator, this unit comprising:
- a stimulation/defibrillation epicardial lead (10) of the screw lead type, including:
. a lead body (12) with a sheath made of a deformable material;
. on the distal side, a lead head with a stimulation/defibrillation electrode and a protruding, helical, anchoring screw (14), adapted to enter into the external wall of the myocardium (34) under the effect of a screwing movement imparted to the lead head; and
. on the proximal side, means for the coupling to the generator of the medical device, and
- a removable guide-catheter (24) for insertion of the epicardial lead, adapted to be introduced in the pericardial space (38),
this guide-catheter comprising a hollow tube, open at its two ends, with a central lumen into which the lead is able to be introduced and moved in translation inside the guide-catheter, first up to a retracted position where the lead head levels with the distal-side outlet of the guide-catheter lumen, then up to an extended position where the lead head, with the anchoring screw, emerges from this outlet, and
the hollow tube of the guide-catheter being a preformed tube, elastically deformable and relatively stiffer than the sheath of the lead body, and showing, in the absence of stress, two successive bends (26, 28) inscribed in two distinct respective surfaces (30, 32),
this unit being **characterized in that**:
- the lead (10) is a monodiameter lead, with the helical anchoring screw (14) continuing axially the lead head;
- said two successive bends (26, 28) are inscribed in a parallelepipedal volume with a size comprised between 15x10x5 mm and 25x15x5 mm;
- the less distal bend (26) of said two successive bends (26, 28) is a bend having an overall size, in the plane, of 15 to 25 mm by 10 to 15 mm,
so as to follow the natural bend of the pericardial space during the progression of the guide-catheter and to provide, once the site of implantation chosen, a bearing of the lead body against the external wall of the pericardial space; and
- the more distal bend (28) of said two successive bends (26, 28) is a bend having an overall size, in elevation, of 10 to 15 mm by 5 mm at most,
so as to allow, once the site of implantation chose, an orientation of the end of the guide-catheter tube according to a predefined attack angle in the direction of the external wall of the myocardium (34), and a holding of the axis (D) of the anchoring screw (14) in this same direction during a combined movement of screwing and displacement of the lead head towards the extended position.

2. The unit of claim 1, wherein the anchoring screw (14) is a fixed screw.

3. The unit of claim 1, wherein the anchoring screw is a mobile screw, retractable into a housing of the lead head.

4. The unit of claim 1, wherein the anchoring screw (14) is an active screw, forming said stimulation/defibrillation electrode.

5. The unit of claim 1, wherein the anchoring screw is a passive screw, electrically decoupled from said stimulation / defibrillation electrode.

6. The unit of claim 1, wherein the sheath of the lead body (12) shows, in torsion, a sufficient stiffness to allow the transmission, over its whole length, of a rotational movement imparted from the proximal end of the lead, for the screwing of the anchoring screw.

7. The unit of claim 1, wherein the sheath of the lead body (12) shows, in torsion, a insufficient stiffness to allow the transmission, over its whole length, of a rotational movement imparted from the proximal end of the lead, and wherein the unit further comprises:
- a removable mandrel, adapted to be introduced into a lumen of the lead body and mobile in translation inside this lumen up to the lead head and including means for the coupling in rotation with the lead head, this mandrel showing, in torsion, a sufficient stiffness to allow the transmission, over its whole length and up to the coupling means, of a rotation movement imparted from the proximal end of the mandrel, for the screwing of the anchoring screw.

8. The unit of claim 1, wherein the diameter of the guide-catheter (24) is lower than or equal to 9 French, preferably lower than or equal to 6 French.

9. The unit of claim 1, wherein the diameter of the lead body (12) is lower than or equal to 5 French.

10. The unit of claim 1, wherein the anchoring screw (14) comprises an end part (20) adapted to enter into the external wall of the myocardium (34), and connected to the lead head by a transition part (22) mechanically deformable in flexion.

11. The unit of claim 10, wherein the transition part (22) mechanically deformable in flexion is a closed-helix part of the helical screw.

12. The unit of claim 1, wherein the anchoring screw comprises an end part (20) adapted to enter into the external wall of the myocardium (34), and itself comprising an electrically conductive active tip region continuing an electrically insulated intermediate part.

13. The unit of claim 12, wherein the axial length of the electrically conductive active tip region is at most of 1 mm.
